# EUROPEAN PATENT SPECIFICATION

(11) **EP 0 845 262 B1**
(45) Date of publication and mention of the grant of the patent: **12.03.2003**
(21) Application number: 97830623.1
(22) Date of filing: 26.11.1997
(51) Int. Cl.: A61K 9/00

(54) **Effervescent propolis compositions**
Brausende Propoliszusammensetzungen
Compositions effervescentes à base de propolis

(30) Priority: 28.11.1996 IT BO960609
(43) Date of publication of application: 03.06.1998
(73) Proprietor: Docteur Nature Srl, 41041 Baggiovara (Modena) (IT)
(72) Inventor: Abram, Raymond, 41041 Baggiovara (Modena) (IT)
(74) Representative: Coppi, Cecilia

(56) References cited:
- DATABASE WPI Section Ch, Week 8634 Derwent Publications Ltd., London, GB; Class B05, AN 86-223474 XP002056487 & RO 88 060 A (INTR. MEDIC BUCUREST) , 28 February 1986
- DATABASE WPI Section Ch, Week 9717 Derwent Publications Ltd., London, GB; Class B05, AN 97-191090 XP002056488 & RU 2 065 278 A (CHIZHOV V P) , 20 August 1996
- DATABASE WPI Section Ch, Week 9434 Derwent Publications Ltd., London, GB; Class D13, AN 94-277223 XP002056489 & RO 106 649 A (ENCIU A) , 30 June 1993

## Description

It is known that effervescent pharmaceutical products are easily available on the market at present in order to make administration easier and quicker whether the drugs are analgesic, vitamin drugs or similar.

This is particularly important when the patient has difficulty swallowing a pill or when a drug must be administered to a child.

It is also known that in cold-caused illnesses, drugs with antipyretic, analgesic and antibiotic effect, which require a further supply of vitamins, are often prescribed.

At present there is no product on the market which combines all the above-mentioned characteristics and therefore more than one drug must be administered.

RU 2065278 discloses a polyvitamin complex in the form of a non-effervescent tablet containing extracts of propolis and dog rose. RO 88060 discloses a therapeutic tablet comprising propolis extract, citric acid and sodium bicarbonate. RO 106649 discloses a carbonated propolis based therapeutic nutritive beverage.

The purpose of this invention is to lead to the production of a natural effervescent product based on propolis, dog rose and white willow.

The properties of these components have long been known.

Propolis is a resin that bees collect from the buds and bark of trees and which is reprocessed by the bees themselves by means of glandular secretions. Even in Ancient Egypt, propolis was already being used to treat various illnesses and the tradition was handed down to the Greeks and Ancient Romans.

The composition of propolis of course varies according to the natural habitat in which the product is first processed and then collected. However the principle substances which are always present include flavonoids, cinnamic acid, amino acids, vitamins of the B group, vitamins of the C and PP groups.

As a consequence, propolis has an anaesthetic, antibacterial, antiviral, antimycotic, cicatrizant, immuno-stimulating effect. Because of these properties it is widely used in dentistry, stomatology, dermatology and otorhinolaryngology.

Dog rose is a wild rose whose flowers produce fruits, named hips, which contain sugars, tannins, organic acids and natural vitamins: carotenoids, flavonoids and vitamin C. The percentage of this latter is very high in the fresh hips: up to 1-2% of the dry weight.

Thanks to these components, the properties of the dog rose make it particularly suitable in treating headaches, rhinopharyngitis, tonsillitis, otitis and asthma caused by allergies.

White willow has a high salicin content, the natural glycoside of salicylic acid which is the active principle of many preparations in the pharmaceutical industry. The antiinflammatory, painkilling and antipyretic properties of salicylic acid are well-known.

In the preparation of effervescent tablets, sodium carbonate or bicarbonate is usually mixed together in the dry state with solid organic acids such as citric acid, for example, or malic acid or similar, and sweeteners, colorants and flavouring and aromatic agents.

When the tablet is placed in water, the carbonate forms carbon dioxide through the action of the acids. thus obtaining a fizzy drink in which the various components are dissolved.

The compound which is the subject of this invention may also be prepared as soluble powder, a liquid to be drunk or capsules using the usual known laboratory techniques.

As an example, an effervescent tablet of 4.500 mg is described, obtained with the usual laboratory techniques, containing:
Propolis 12.500 mg.
White willow 25.000 mg.
Dog rose 12.500 mg.
Citric acid 2.360 mg.
Sodium bicarbonate 1693.5 mg.
combined with sweeteners, starch, aromatic agents and colorants.

## Claims

1. NATURAL COMPOUND WITH ANTIBIOTIC AND ANALGESIC EFFECT, **characterised in that** it comprises of extract of propolis mixed with an acid, a carbonate or bicarbonate to generate carbon dioxide when in contact with water, further including extract of white willow and dog rose, and being in the form of an effervescent tablet, a soluble powder or capsules; said forms of production being obtained in accordance with known laboratory techniques.

2. NATURAL COMPOUND as described in claim 1, **characterised in that** its components include sweeteners, colorants and aromatic agents.

## Patentansprüche

1. NATÜRLICHE VERBINDUNG MIT ANTIBIOTISCHER UND SCHMERZSTILLENDER WIRKUNG, **dadurch gekennzeichnet, dass** sie einen Auszug aus Propolis vermischt mit einem Acidum, einem Karbonat oder einem Bikarbonat enthält, um bei Kontakt mit Wasser ein Kohlendioxid zu erzeugen und sie darüberhinaus Auszüge der Silberweide und aus Hagebutte enthält und in Form von Brausetabletten, auflösbarem Pulver oder Kapseln präsentiert wird. Diese Produktionsformen werden in Übereinstimmung mit den bekannten Labortechniken hergestellt.

2. NATÜRLICHE VERBINDUNG nach Anspruch 1, **dadurch gekennzeichnet, dass** ihre Komponenten Süßstoffe, Farbstoffe und Aromastoffe enthalten.

## Revendications

1. COMPOSÉ NATUREL AYANT UN EFFET ANTIBIOTIQUE ET ANALGESIQUE **caractérisé en ce qu'**il contient de l'extrait de propolis mélangé à un acide, un carbonate ou bicarbonate pour générer du dioxyde de carbone lorsqu'il entre en contact avec de l'eau, contenant également de l'extrait de saule blanc et de rosier sauvage, sous forme de cachet effervescent, de poudre soluble ou de capsules, de telles formes de production étant obtenues conformément aux techniques de laboratoire connues.

2. COMPOSÉ NATUREL selon la revendication 1 **caractérisé en ce que** ses composants contiennent des édulcorants, des colorants et des arômes.
